# DEMANDE DE BREVET EUROPEEN

(11) **EP 1 990 061 A2**
(43) Date de publication de la demande: **12.11.2008**
(21) Numéro de dépôt: 08160581.8
(22) Date de dépôt: 19.02.2004
(51) Int. Cl.: A61K 47/02, A61K 47/12, A61K 47/14

(54) **Composition pharmaceutique pour administration transdermique ou transmuqueuse**

(30) Priorité: 20.02.2003 FR 0302083; 12.05.2003 US 436380
(62) Demande divisionnaire de: 04712591.9
(71) Demandeur: Besins International Belgique, 1620 Drogenbos (BE)
(72) Inventeur: Taravella, Brigitte, 75013, PARIS (FR); Masini-Eteve, Valérie, 91370, VERRIERE LE BUISSON (FR)
(74) Mandataire: Cabinet Plasseraud

(57) **Abrégé**

La présente invention a pour objet une nouvelle composition pharmaceutique destinée à être administrée par voie transdermique ou par voie transmuqueuse d'au moins une substance active, et comprenant notamment un acide gras en tant que promoteur d'absorption percutanée, au moins un véhicule alcoolique, ainsi qu'un agent stabilisant capable de stabiliser l'acide gras dans ladite composition pharmaceutique.

## Description

La présente invention a pour objet une nouvelle composition pharmaceutique destinée à être administrée par voie transdermique ou transmuqueuse d'au moins une substance active, et comprenant notamment un acide gras en tant que promoteur d'absorption percutanée, ainsi qu'un agent stabilisant capable de stabiliser l'acide gras dans ladite composition pharmaceutique.

Il est bien connu que certaines substances actives ne sont pas adaptées à l'administration par voie orale pour des raisons variables qui sont entre autres liées soit à un fort métabolisme hépatique : « effet de 1^{er} passage » ; soit à une forte dégradation gastro-intestinale.

Des formulations transdermiques ou transmuqueuses ont ainsi été développées afin de contourner ces inconvénients.

Les compositions pharmaceutiques destinées à être administrées par voie transdermique ou par voie transmuqueuse possèdent en effet plusieurs avantages par rapport à des formes orales : élimination du problème de métabolisme par le foie de la substance active, pas de dégradation gastrique de la substance active, effet réservoir possible avec une libération continue du principe actif dans le temps. Elles peuvent cependant poser des problèmes au niveau du passage des substances actives à travers la peau.

En effet, toutes les substances actives ne diffusent pas de façon similaire à travers la barrière percutanée. Cette diffusion est fonction des caractéristiques chimiques et physico-chimiques de la molécule.

Afin de faciliter ce passage des substances actives à travers la peau, les formulations transdermiques ou transmuqueuses peuvent inclure des molécules spécifiques nommées « promoteur d'absorption percutanée ». Cette désignation concerne toute molécule favorisant la diffusion d'un principe actif à travers la peau ou la muqueuse de façon réversible, et tout solubilisant favorisant le partage du principe actif entre le véhicule et la couche cornée de l'épiderme ou de la muqueuse. Une des classes de promoteurs d'absorption couramment mis en oeuvre dans les formulations transdermiques ou transmuqueuses est la classe des acides gras.

La Société Demanderesse a cependant montré que des compositions hydroalcooliques contenant ces acides gras n'étaient pas stables d'un point de vue chimique. En effet, l'acide gras, de part sa fonction « acide » et en présence d'alcool, subit selon les lois de la chimie organique, une réaction d'estérification. Ainsi, par exemple, l'acide oléique se transforme en oléate d'éthyle en présence d'éthanol.

Cependant, les médicaments étant souvent stockés un certain temps avant leur vente, il est essentiel que leur composition soit invariable afin d'assurer une sécurité au niveau de la santé des patients. Tout médicament doit donc démontrer une haute stabilité, c'est-à-dire que sa composition ne doit pas varier avec le temps. Une stabilité de la composition pharmaceutique est une garantie de constance en terme de biodisponibilité de l'actif, d'efficacité de la composition pharmaceutique, d'acceptabilité et d'innocuité du médicament pour le patient.

Ainsi, dans un gel ou une solution hydroalcoolique, la dégradation de l'acide gras, promoteur de l'absorption percutanée de la substance active, peut si elle est significative modifier considérablement la biodisponibilité de la substance active et donc son efficacité.

La Société Demanderesse a conduit de nombreux travaux et recherches afin d'apporter une solution à ce problème de stabilité des acides gras en milieu alcoolique, pour obtenir une stabilité satisfaisante.

Elle a ainsi trouvé qu'il était possible de stabiliser l'acide gras dans la composition pharmaceutique en ajoutant un tampon et/ou en ajoutant de petites quantités d'un ester de l'acide gras correspondant, dès la préparation de ladite composition pharmaceutique.

L'invention concerne donc une composition pharmaceutique destinée à l'administration par voie transdermique ou par voie transmuqueuse d'au moins une substance active, et comprenant au moins un acide gras en tant que promoteur d'absorption percutanée, au moins un véhicule alcoolique, ainsi qu'au moins un agent stabilisant capable de stabiliser l'acide gras dans ladite composition pharmaceutique.

Dans le contexte de la présente invention on entend par « stable » une composition pharmaceutique dont la composition qualitative et quantitative, ainsi que les caractéristiques physiques, chimiques et biologiques n'évoluent pas de façon significative au cours du temps dans des conditions de température et d'humidité données, soit pendant 3 ans à 25°C/60%HR, pendant 1 an à 30°C/65%HR et/ou pendant 6 mois à 40°C/75%HR.

On entend par « évolution significative » tout changement qualitatif et/ou quantitatif, ainsi que tout changement des caractéristiques physiques, chimiques et biologiques qui se produit en dehors des critères d'acceptation définis pour la méthode analytique mise en oeuvre pour chaque test chimique, physique ou biologique (limite numérique, intervalle, ou autre mesure pertinente).

Le ou les promoteurs d'absorption percutanée entrant dans la composition pharmaceutique selon l'invention sont de préférence sélectionnés dans le groupe constitué par les acides gras saturés ou insaturés.

Il s'agit de préférence des acides gras aliphatiques à longues chaînes contenant de 10 à 18 atomes de carbone. Les acides gras sont sélectionnés, de façon non limitative, dans le groupe constitué par l'acide caprique (10:0), l'acide laurique (12:0), l'acide myristique (14:0), l'acide palmitique (16:0), l'acide stéarique (18:0) ; l'acide oléique (18:1), l'acide palmitoléique (16:1), l'acide linoléique (18:2), et l'acide linolénique (18:3).

La teneur en acide gras dans la composition pharmaceutique selon la présente invention sera avantageusement comprise entre 0,1% et 20%, de préférence entre 0,2% et 10%, et plus préférentiellement encore entre 0,5% et 5%, ces pourcentages étant exprimés en poids par rapport à 100g de composition pharmaceutique.

Cependant, il est explicitement précisé ici que la composition pharmaceutique selon l'invention peut contenir d'autres promoteurs d'absorption percutanée en combinaison avec le ou les acides gras.

L'agent stabilisant est sélectionné dans le groupe constitué par les tampons et/ou par les esters des acides gras correspondants aux acides gras présents dans la composition pharmaceutique en tant que promoteurs d'absorption percutanée.

Le ou les tampons, lorsqu'ils sont présents dans la composition pharmaceutique selon l'invention, permettent de maintenir avantageusement le pH de ladite composition entre 4 et 10, de préférence entre 5 et 9 et plus préférentiellement encore entre 6 et 8.

La teneur en tampon(s) de la composition pharmaceutique selon l'invention est avantageusement comprise entre 1% et 80%, de préférence entre 5% et 70% et plus préférentiellement encore entre 10% et 50%, ces pourcentages étant exprimés en poids par rapport à 100g de composition pharmaceutique.

Selon un mode de réalisation préféré de la composition pharmaceutique selon l'invention, les tampons sont sélectionnés dans le groupe constitué par :
- - les tampons alcalinisants ou basiques tels qu'un tampon phosphate (phosphate de sodium dibasique ou monobasique par exemple), un tampon citrate (citrate de sodium ou citrate de potassium par exemple), le carbonate de sodium, le bicarbonate de sodium, étant de préférence un mélange de carbonate de sodium et de bicarbonate de sodium, ou
- - les tampons neutres tels qu'un tampon Tris, de préférence un tampon phosphate.

Selon un autre mode de réalisation de la composition pharmaceutique selon l'invention, le ou les esters d'acides gras saturés ou insaturés mis en oeuvre sont ceux qui résulteraient de la réaction entre un acide gras identique à celui contenu dans la dite composition avec un alcool identique à celui contenu dans la dite composition. Les esters d'acide gras sont donc sélectionnés, de préférence, dans le groupe constitué par l'oléate d'éthyle, l'oléate d'isopropyle, le myristate d'isopropyle, le palmitate d'isopropyle, l'octanoate d'éthyle, le dodecanoate d'éthyle, le linoléate d'éthyle, le linolénate d'éthyle.

La teneur en ester d'acide gras ajouté en tant qu'agent stabilisant dans la composition pharmaceutique selon l'invention est avantageusement comprise entre 0,1% et 10%, de préférence entre 0,2% et 5% et plus préférentiellement encore entre 0,5% et 2,5%, ces pourcentages étant exprimés en poids par rapport à 100g de composition pharmaceutique.

La composition pharmaceutique selon l'invention contient également un véhicule non aqueux de type alcool. Le véhicule alcoolique doit être capable de dissoudre l'ensemble des composants de la formulation et notamment l'acide gras et éventuellement son ester. De préférence, le véhicule alcoolique peut être sélectionné parmi l'éthanol et/ou l'isopropanol. Toutefois, l'éthanol représente un véhicule préféré selon l'invention puisqu'il contribue avec efficacité au passage transcutané du principe actif en s'évaporant rapidement au contact avec la peau.

De façon avantageuse la teneur en alcool est comprise entre 10% et 90%, de préférence entre 20% et 80%, et plus préférentiellement encore entre 40% et 70%, ces pourcentages étant exprimés en poids par rapport à 100g de composition pharmaceutique.

La composition pharmaceutique selon l'invention peut également comprendre un véhicule aqueux. Le véhicule aqueux permet de solubiliser les molécules hydrophiles contenues dans la formulation et favorise également la diffusion des molécules lipophiles de la formulation vers la couche cornée. Elle forme un véhicule binaire avec le solvant non aqueux.

Le véhicule aqueux sera préférentiellement de l'eau. Sa teneur est comprise entre 1% et 80%, de préférence entre 10% et 70%, et plus préférentiellement encore entre 20% et 60%, ces pourcentages étant exprimés en poids par rapport à 100g de composition pharmaceutique.

La composition pharmaceutique selon l'invention peut également contenir un agent co-solvant tel que les polyols ou polyglycols comme par exemple le glycérol (ou glycérine), le propylèneglycol ou le polyéthylèneglycol pour une teneur comprise entre 0,5% et 20%, de préférence entre 1% et 10%, ces pourcentages étant exprimés en poids par rapport à 100 g de composition pharmaceutique. L'agent co-solvant permet d'augmenter la solubilité des substances actives.

La composition pharmaceutique selon l'invention peut se présenter sous la forme d'un gel, d'une solution, d'une crème, d'une lotion, d'un lait, d'une pommade, d'un aérosol, ou d'un patch.

De préférence, elle se présente sous la forme d'un gel ou d'une solution.

Lorsque la composition pharmaceutique selon l'invention se présente sous la forme d'un gel, elle comprend également un agent gélifiant.

D'une manière avantageuse, et selon le type d'agent gélifiant mis en oeuvre, elle présente une teneur comprise entre 0,2% et 30% d'un agent gélifiant, de préférence entre 0,5% et 10% et plus préférentiellement encore entre 0,3% et 5%, ces pourcentages étant exprimés en poids pour 100g de composition pharmaceutique.

Les carbomères ou acides polyacryliques tels que le carbopol 980 ou 940 NF, 981 ou 941 NF, 1382 ou 1382 NF, 5984, 2984 ou 934 NF, Pemulen TR1 NF ou TR2 NF, Ultrez, le Synthalen CR, les polymères acryliques non pré-neutralisés ; les dérivés cellulosiques tels que l'éthylcellulose, l'hydroxypropyl cellulose, l'hydroxyethyl cellulose, l'hydroxypropylméthyle cellulose (HPMC), la carboxyméthyle cellulose (CMC), etc ; les poloxamères ou polyéthylène-polypropylène copolymères tel que le Lutrol F grade 68 ou 127, les poloxamines ou d'autres agents gélifiants tels que le chitosan, le dextran, les pectines, les gommes naturelles, seuls ou en association peuvent être utilisés en tant qu'agents gélifiants dans la composition pharmaceutique selon l'invention.

Ces agents gélifiants permettent d'augmenter la viscosité des formulations selon l'invention mais peuvent également jouer le rôle d'agent de solubilisation.

Le Carbopol® 980, et/ou l'hydroxypropylcellulose sont particulièrement préférés dans le cadre de la présente invention.

Le choix de l'agent gélifiant sera réalisé en fonction du pH de la composition définie selon l'invention et de la viscosité recherchée.

Selon un autre mode de réalisation avantageux de la composition pharmaceutique selon l'invention, en présence de certains types de gélifiants, et notamment les polymères acryliques non pré-neutralisés, elle peut contenir un agent neutralisant. Le rapport neutralisant/gélifiant est alors compris entre 10/1 et 0,1/1, de préférence entre 7/1 et 0,5/1, et plus préférentiellement encore entre 4/1 et 1/1.

L'agent neutralisant est choisi de telle manière qu'il forme en présence du polymère des sels qui soient solubles dans le véhicule.

L'agent neutralisant est également choisi de façon à permettre d'atteindre un gonflement optimal des chaînes de polymère lors de la neutralisation des charges et de la formation de sels de polymères.

Selon l'invention, la triéthanolamine et/ou la trométhamine (2-Amino 2-Hydroxyméthyle-1, 3-propanediol) sont utilisées de préférence comme agents neutralisants en présence de Carbopol® 980. Elles permettent également d'atteindre une viscosité optimale dans la composition pharmaceutique selon l'invention.

D'autres agents neutralisants comme l'hydroxyde de sodium, l'hydroxyde d'ammonium, l'hydroxyde de potassium, l'arginine, l'aminométhyle de propanol peuvent être utilisés dans la composition pharmaceutique selon l'invention. L'agent neutralisant est choisi en fonction du type de gélifiant utilisé, d'une manière connue par l'homme du métier.

La ou les substance(s) active(s) présente(s) dans la composition pharmaceutique selon l'invention peut (peuvent) avantageusement être sélectionnée(s) dans le groupe constitué par les oestrogènes, les progestatifs, les androgènes, les anti-oestrogènes, les anti-androgènes ou leurs mélanges.

De façon avantageuse la teneur en substance active est comprise entre à 0,01% et 5%, ces pourcentages étant exprimés en poids par rapport à 100g de composition pharmaceutique.

Les anti-oestrogènes peuvent être sélectionnés dans le groupe constitué par le tamoxifène, le 4-hydroxytamoxifène, le citrate de tamoxifène, le toremifène, le droloxifène, et le raloxifène.

Les androgènes peuvent être sélectionnés dans le groupe constitué par l'androgène naturel, la testostérone, et ses dérivés semi-naturels ou synthétiques comme la méthyltestostérone ; les précurseurs physiologiques de la testostérone tels que la déhydroépiandrostérone ou DHEA ou encore la prastérone et ses dérivés comme le sulfate de DHEA, la Δ-4-androstènedione et ses dérivés ; les métabolites de la testostérone comme la dihydrotestostérone (DHT) obtenue après l'action enzymatique des 5-α-réductases ; ou bien des substances possédant un effet de type androgénique tel que la tibolone.

Les anti-androgènes peuvent être sélectionnés dans le groupe constitué par les composés stéroïdiens tels que l'acétate de cyprotérone, la médroxyprogesterone, ou par les composés non stéroïdiens tels que le flutamide, le nilutamide ou le bicalutamide. De préférence, la substance active dans la composition pharmaceutique selon l'invention est un progestatif, un oestrogène ou une combinaison des deux.

Avantageusement, le ou les progestatifs mis en oeuvre dans la composition pharmaceutique selon l'invention peuvent êtres sélectionnés dans le groupe constitué par les progestatifs naturels, la progestérone ou ses dérivés de type ester, les progestatifs de synthèse de type 1, 2 ou 3. Le premier groupe comprend les molécules proches de la progestérone ou les progestatifs de synthèse 1 (PS1) (pregnanes), par exemple, l'isomère de progestérone (rétroprogestérone), le Medrogestérone, les dérivés de la norprogestérone (démégestone ou promégestone). Le second groupe comprend les dérivés de la 17α-hydroxy-pragestérane ou progestatifs de synthèse 2 (PS2) (pregnanes), par exemple l'acétate de cyprotérone, l'acétate de médroxyprogestérone. Le troisième groupe comprend les norstéroides ou progestatifs de synthèse 3 (PS3), (estranes ou nor-androstanes). Ce sont des dérivés de la 19-nortestostérone, par exemple la noréthindrone. Ce groupe comprend également les molécules de type gonane qui dérivent de ces nor-androstanes ou estranes et présentent un groupe méthyle en C18 et un groupe éthyle en C13. On peut citer comme exemples le norgestimate, le désogestrel (3-cétodésogestrel) ou le gestodène.

La tibolone, qui présente à la fois une activité progestative et androgénique peut être également avantageusement sélectionnée dans la composition pharmaceutique selon l'invention.

Le ou les oestrogènes mis en oeuvre dans la composition pharmaceutique selon l'invention peuvent avantageusement êtres sélectionnés dans le groupe constitué par les oestrogènes naturels : le 17-β oestradiol, l'oestrone, les oestrogènes conjugués équins, l'estriol, les phytoestrogènes; par les oestrogènes semi-naturels : le valérate d'oestradiol ; ou bien par les oestrogènes synthétiques : l'éthinyl-estradiol ; de préférence, le 17-β estradiol.

Selon un mode de réalisation avantageux de la composition pharmaceutique selon l'invention, la teneur en progestatif(s) sera comprise entre 0,01% et 5%, de préférence entre 0,02% et 3%, et plus préférentiellement encore entre 0,03% et 1%, ces pourcentages étant exprimés en poids par rapport à 100g de composition pharmaceutique.

Selon un autre mode de réalisation particulier de la composition pharmaceutique selon l'invention, la teneur en oestrogène(s) sera comprise entre 0,01% et 5%, de préférence entre 0,02% et 3%, et plus préférentiellement encore entre 0,03% et 2%, ces pourcentages étant exprimés en poids par rapport à 100g de composition pharmaceutique.

L'invention sera mieux comprise à l'aide des exemples non limitatifs décrits ci-dessous.

### EXEMPLE 1 : INSTABILITE DE GELS COMPRENANT L'ACIDE OLEIQUE

Une première série de gels ayant les compositions données au Tableau I a été soumise à des essais de stabilité.

La fabrication d'un gel selon l'invention se fait comme suit :
Soit, pour 40 kg d'un gel fabriqué selon la formulation E760 contenant deux principes actifs, l'Estradiol (Diosynth)et la Progestérone (Xianming) :
   - - Dans une cuve mélangeur de type Koruma, on introduit sous vide de 800 mb 26000 g d'éthanol 95°.
   - - Puis, par le dessus de la cuve, on charge sans agiter, successivement, 2000 g de propylène glycol, 1200 g d'acide oléique. On mélange l'ensemble durant au moins 5 minutes, turbine à 2000 trs/min, racleur à 40 trs/min.
   - - On ajoute 24,8 g d'Estradiol. Puis, on mélange 15 minutes, turbine à 2000 trs/min, racleur à 40 trs/min.
   - - On ajoute 800 g de Progestérone. Puis, on mélange 15 minutes, turbine à 2000 trs/min, racleur à 40 trs/min.
   - - On introduit 400 g de Klucel®HF sous vide 800 mb, turbine à 1500 trs/min.
   - - On agite 10 minutes au moins, turbine à 1500 trs/min, racleur à 40 trs/min.
   - - Puis, on charge l'eau purifiée en quantité suffisante pour obtenir une masse totale de 40000 g, sous vide 800 mb, racleur à 40trs/min.
   - - On mélange pendant 10 minutes au moins, turbine à 1500 trs/min, racleur à 40 trs/min.
   - - On arrête la turbine.
   - - On fait le vide à 100 mb pendant environ 2 minutes.
   - - On agite lentement pendant au moins 10 minutes, racleur à 10 trs/min.

Les autres formulations sont préparées de la même manière en modifiant les quantités des différents composants selon le tableau I.

Les taux d'acide oléique et d'oléate d'éthyle ont été mesurés respectivement par chromatographie en phase gazeuse ou par potentiométrie (et par potentiométrie) après stockage à température ambiante (25°C) et 60% d'humidité relative aux temps : 0, 1, 2, 3, 6, 10, 12 et 18 mois (et 0, 1, 3, 4, 6, 12, 14 et 20 mois pour le placebo).

Les résultats sont donnés dans les tableaux II et III.

Les spécifications ou normes inscrites dans les tableaux correspondent au critère d'acceptation fixé qui est de plus ou moins 5% par rapport à la valeur théorique initiale. Toute valeur située en dehors de cet intervalle d'acceptabilité est jugée non conforme et correspond à une variation dite « significative ». Ces valeurs apparaissent en grisées dans les tableaux.

**Tableau I : Composition des gels hydroalcooliques testés**

| | **Echantillons** | | | | |
|---|---|---|---|---|---|
| | **E751 placebo** | **E760** | **E761** | **E762** | **E763** |
| **Progestérone en%** | 0 | 2 | 2 | 3 | 3 |
| **Estradiol en%** | 0 | 0,06 | 0,06 | 0,06 | 0,06 |
| **Propylèneglycol en%** | 5 | 5 | 5 | 5 | 5 |
| **Acide oléique en%** | 2 | 3 | 3 | 2 | 2 |
| **Klucel®* en%** | 1,5 | 1 | 1,5 | 1 | 1, 5 |
| **EtOH 95% v/v** | 65 | 65 | 65 | 65 | 65 |
| **Eau purifiée** | Qsp 100g | Qsp 100g | Qsp 100g | Qsp 100g | Qsp 100g |

| | | | | | |
|---|---|---|---|---|---|
| * Hydroxypropylcellulose fabriqué par la société Aqualon. | | | | | |

Un essai de vieillissement accéléré a également été réalisé sur les mêmes gels qui ont été stockés à une température de 40°C, avec une humidité relative de 75% aux temps 0, 1, 2 , 3, 4, 5, 6, 10, 11, 12, 18 mois. Les taux d'acide oléique et d'oléate d'éthyle ont ensuite été mesurés comme précédemment, aux mêmes temps. Les résultats sont donnés dans le tableau IV.

Une estérification significative de l'acide oléique est observée dans tous les gels au cours du vieillissement. Le taux d'oléate d'éthyle formé et la vitesse d'estérification sont d'autant plus élevés que le pourcentage d'acide oléique est grand au départ et que les conditions de température et d'humidité sont élevées (différence entre 25°C/60%HR et 40°C/75%HR).

Cette estérification de l'acide oléique pose deux soucis majeurs relatifs à l'intégrité de la spécialité pharmaceutique:
- - l'oléate d'éthyle est peu soluble dans l'alcool, ce qui peut entraîner une émulsion non souhaitée dans le gel ;
- - l'acide oléique ayant été sélectionné pour son rôle de promoteur d'absorption percutanée, une diminution de son taux entraînera une incidence néfaste sur le passage percutané des substances actives.

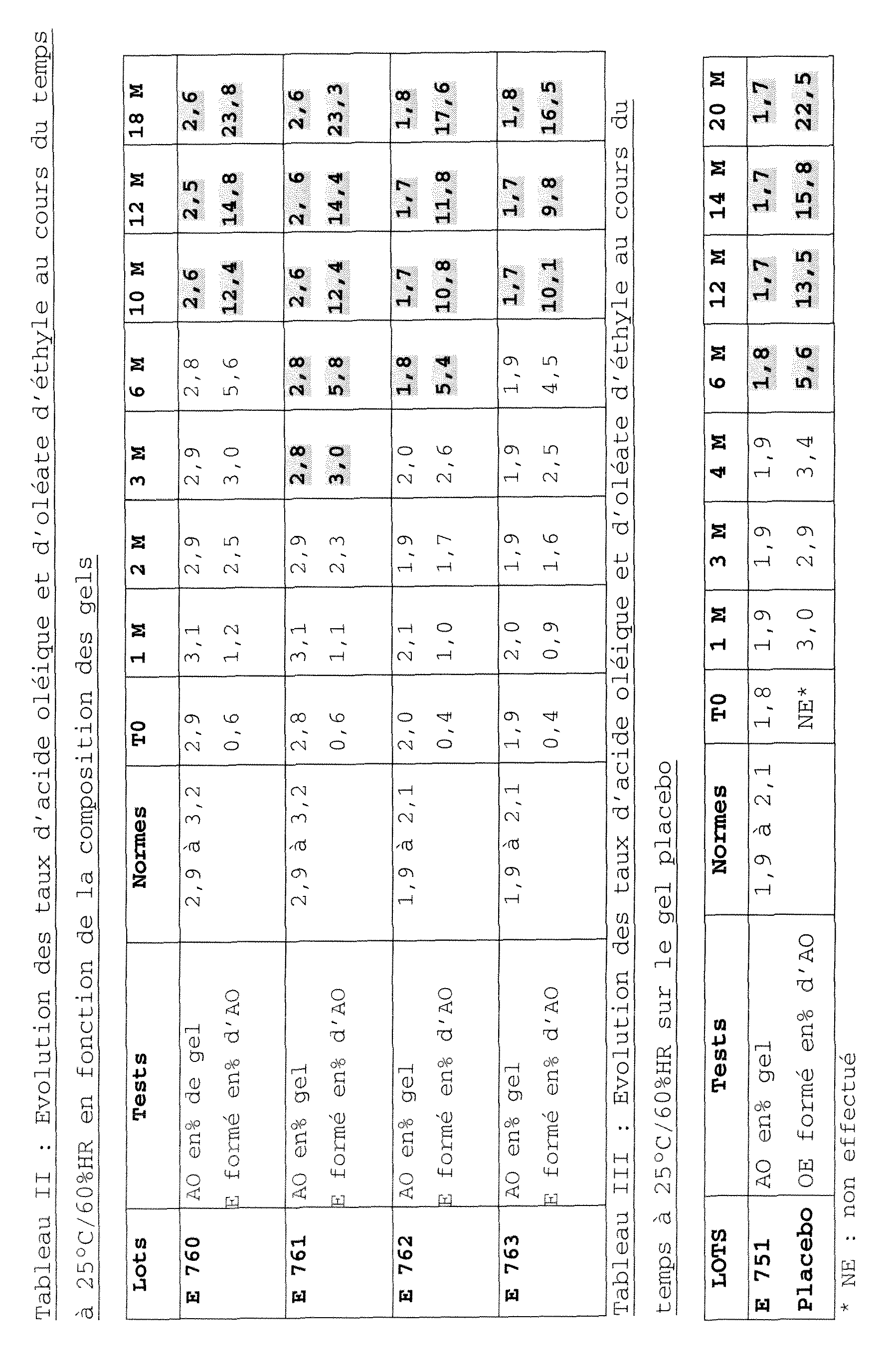

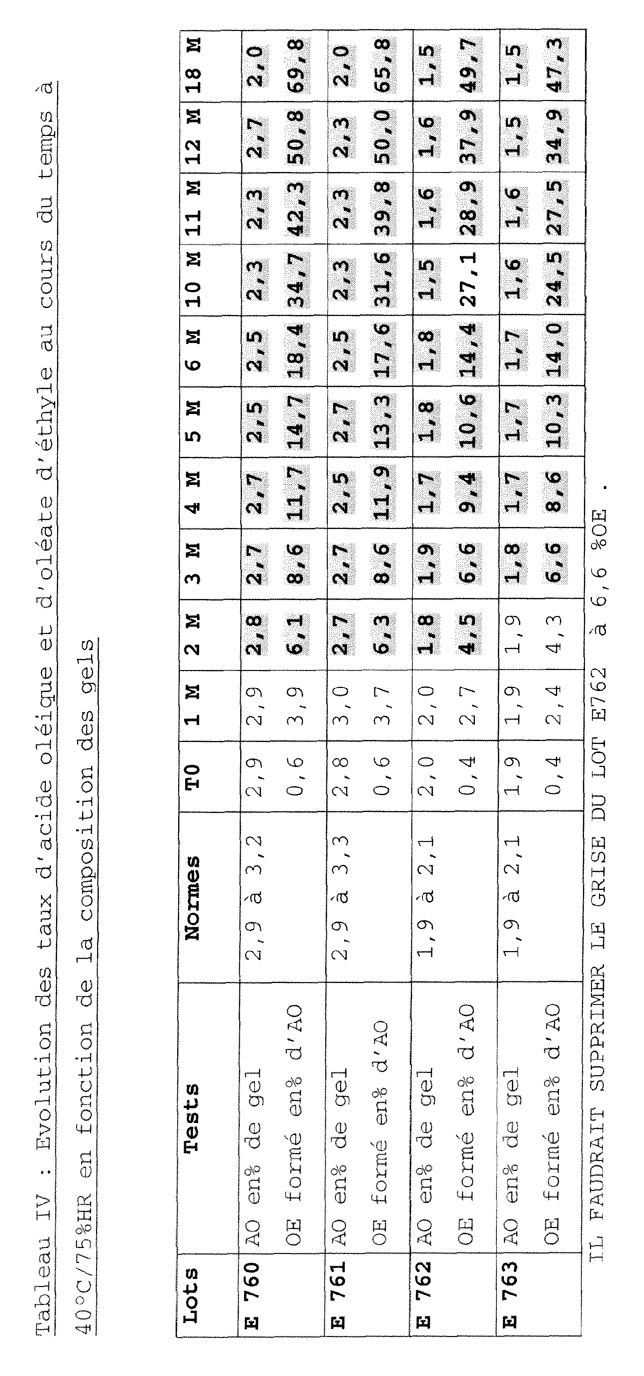

### EXEMPLE 2 : INSTABILITE DE GELS COMPRENANT L'ACIDE OLEIQUE ET LEUR STABILISATION SELON L'INVENTION

Une deuxième série de gels ayant les compositions données au Tableau V a été soumise à des essais de stabilité.

La fabrication d'un gel selon l'invention se fait comme suit :
- - Soit, pour 30 kg d'un gel fabriqué selon la formulation E844 contenant deux principes actifs, l'Estradiol (Diosynth) et la Progestérone (Xianming) :
- - Dans une cuve mélangeur de type Koruma, on introduit sous vide 800 mb 19500 g d'éthanol 95°.
- - Puis, par le dessus de la cuve, on charge sans agiter, successivement, 1500 g de propylène glycol, 1500 g d'acide oléique. On mélange l'ensemble durant au moins 5 minutes, turbine à 2000 trs/min, racleur à 40 trs/min.
- - On ajoute 18,6 g d'Estradiol. Puis, on mélange 15 minutes, turbine à 2000 trs/min, racleur à 40 trs/min.
- - On ajoute 600 g de Progestérone. Puis, on mélange 15 minutes, turbine à 2000 trs/min, racleur à 40 trs/min.
- - On introduit 300 g de Klucel®HF sous vide 800 mb, turbine à 1500 trs/min.
- - On agite 10 minutes au moins, turbine à 1500 trs/min, racleur à 40 trs/min.
- - Puis, on charge l'eau purifiée en quantité suffisante pour 30000 g, sous vide 800 mb, racleur à 40 trs/min.
- - On mélange pendant 10 minutes au moins, turbine à 1500 trs/min, racleur à 40 trs/min.
- - On arrête la turbine.
- - On fait le vide à 100 mb pendant environ 2 minutes.
- - On agite lentement pendant au moins 10 minutes, racleur à 10 trs/min.

Les autres formulations E 845 à E 854 ont été préparées de la même manière en modifiant les quantités de composants introduits selon le Tableau V.

Les échantillons E858 à E865 contiennent un tampon Carbonate-Bicarbonate pH 10,7. Ce tampon est chargé à la place de l'eau purifiée lors de la préparation du gel selon l'invention. Il a été préparé de la façon suivante :
- - Une solution dans l'eau purifiée de Na₂CO₃ anhydre 0,2M, soit : 21,2 g/l (Solution A) a été préparée.
- - Une solution dans l'eau purifiée de NaHCO₃ 0,2M, soit : 16,8 g/l (solution B) a été préparée.
- - Puis 21,3 ml de la solution A ont été ajoutés à 3,8 ml de solution B et de l'eau a été ajoutée pour obtenir 100 ml. La solution résultante correspond au tampon utilisé dans les essais E858 à E 865. Le pH de cette solution tampon est de 10,7.

Les spécifications ou normes inscrites dans les tableaux correspondent au critère d'acceptation fixé qui est de plus ou moins 5% par rapport à la valeur théorique initiale. Toutes valeurs situées en dehors de cet intervalle d'acceptabilité est jugée non conforme et correspond à une variation dite « significative ». Ces valeurs apparaissent en grisées dans les tableaux.

Les gels ont été stockés à 25°C et 60% d'humidité relative et à 40°C et 75% d'humidité relative (vieillissement accéléré). Les taux d'acide oléique et d'oléate d'éthyle ont été mesurés comme précédemment, aux temps : 0, 1, 2, 3 et 6 mois. Les résultats sont donnés dans le tableau VI (25°C/60%HR) et VII (40°C/75%HR).

Ces analyses démontrent que l'utilisation du tampon Carbonate-Bicarbanate à pH 10,7 réduit de façon significative l'estérification de l'acide oléique en oléate d'éthyle.

**Tableau V : Composition des gels hydroalcooliques E844 à 865**

| | **Progestérone %** | **Estradiol %** | **Polypropylène Glycol %** | **Acide oléique %** | **KLUCEL^{a} %** | **ETOH 95% V/V %** | **Isopropanol %** | **qsp 100 g** |
|---|---|---|---|---|---|---|---|---|
| **E 844** | 2 | 0,06 | 5 | 5 | 1 | 65 | 0 | Eau |
| **E*845** | 2 | 0,06 | 5 | 5 | 1 | 65 | 0 | Eau |
| **E 846** | 3 | 0,06 | 5 | 5 | 1 | 65 | 0 | Eau |
| **E*847** | 3 | 0,06 | 5 | 5 | 1 | 65 | 0 | Eau |
| **E 848** | 3 | 0,06 | 5 | 5 | 1,5 | 65 | 0 | Eau |
| **E*849** | 3 | 0,06 | 5 | 5 | 1,5 | 65 | 0 | Eau |
| **E 850** | 3 | 0,06 | 5 | 2 | 1 | 65 | 0 | Eau |
| **E*851** | 3 | 0,06 | 5 | 2 | 1 | 65 | 0 | Eau |
| **E 852** | 2 | 0,06 | 5 | 5 | 1,5 | 65 | 0 | Eau |
| **E*853** | 2 | 0,06 | 5 | 5 | 1,5 | 65 | 0 | Eau |
| **E 854** | 3 | 0,06 | 5 | 2 | 1,5 | 65 | 0 | Eau |
| **E*855** | 3 | 0,06 | 5 | 2 | 1,5 | 65 | 0 | Eau |
| **E 858** | 2 | 0,06 | 5 | 5 | 1 | 65 | 0 | **Tampon** |
| **E*859** | 2 | 0,06 | 5 | 5 | 1 | 65 | 0 | **Tampon** |
| **E 861** | 3 | 0,06 | 5 | 2 | 1 | 65 | 0 | **Tampon** |
| **E*862** | 3 | 0,06 | 5 | 2 | 1 | 65 | 0 | **Tampon** |
| **E 863** | 3 | 0,06 | 5 | 5 | 1 | 65 | 0 | **Tampon** |
| **E*864** | 3 | 0,06 | 5 | 5 | 1 | 65 | 0 | **Tampon** |
| **E 865** | 5 | 0,06 | 5 | 3 | 1 | 30 | 35 | **Tampon** |

| | | | | | | | | |
|---|---|---|---|---|---|---|---|---|
| ^{a} Hydroxypropylcellulose fabriqué par la société Aqualon. * Fournisseur = SCHERING - Pour les autres lots,fournisseur = DIOSYNTH | | | | | | | | |

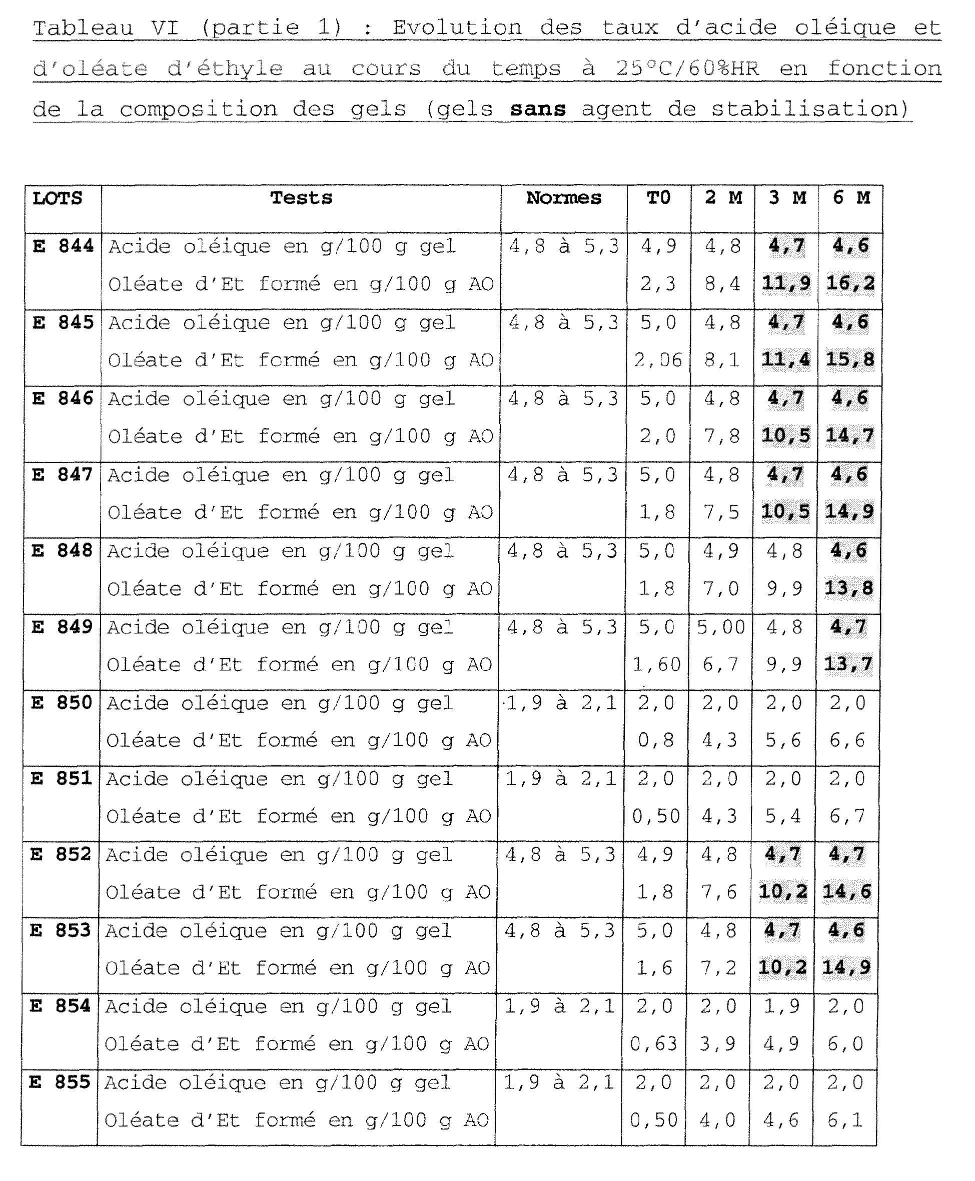

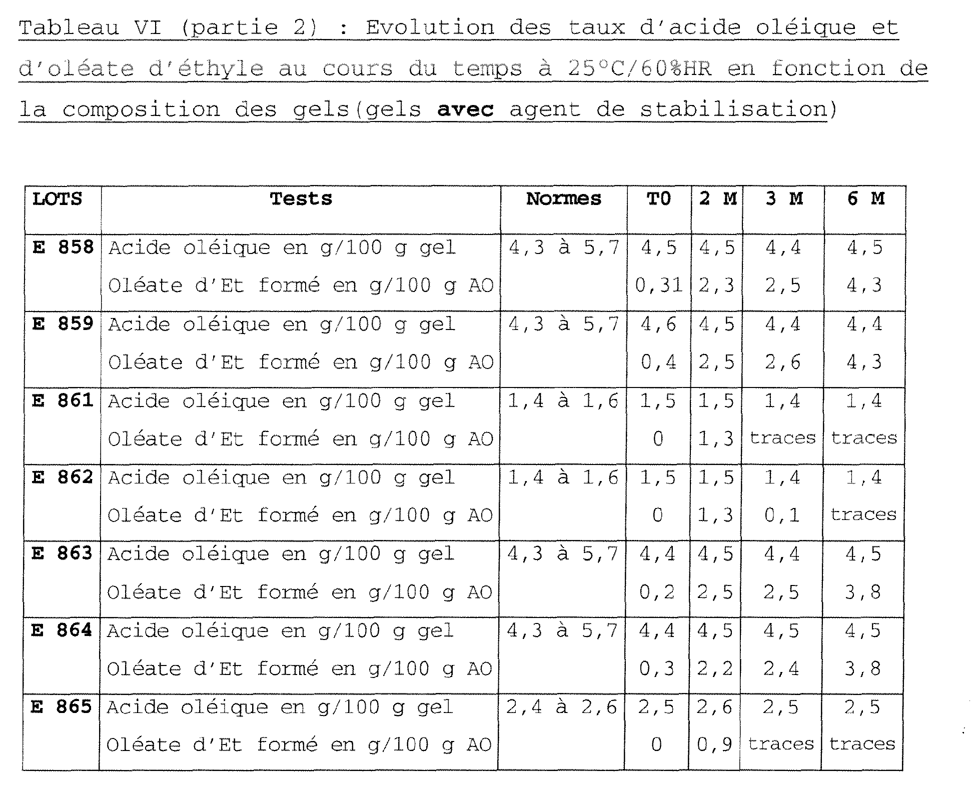

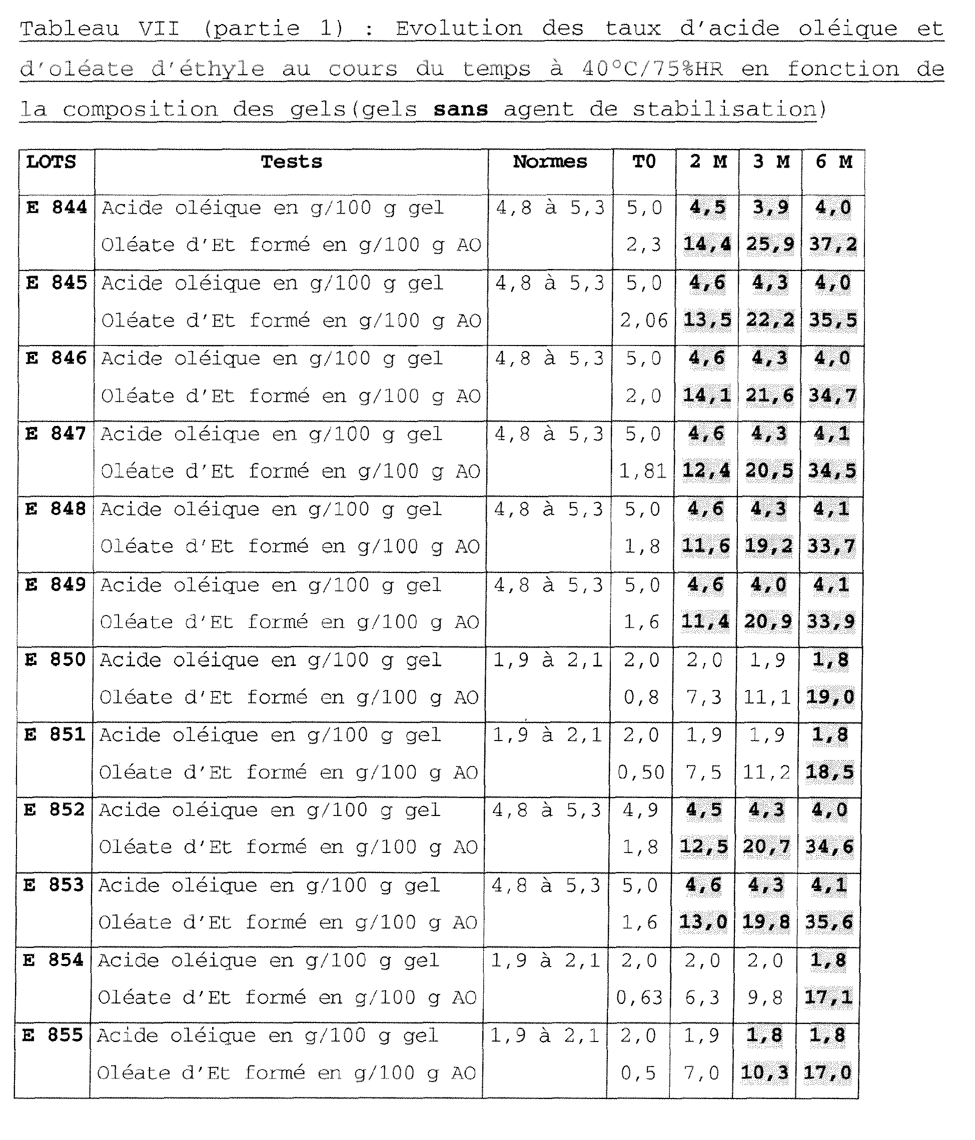

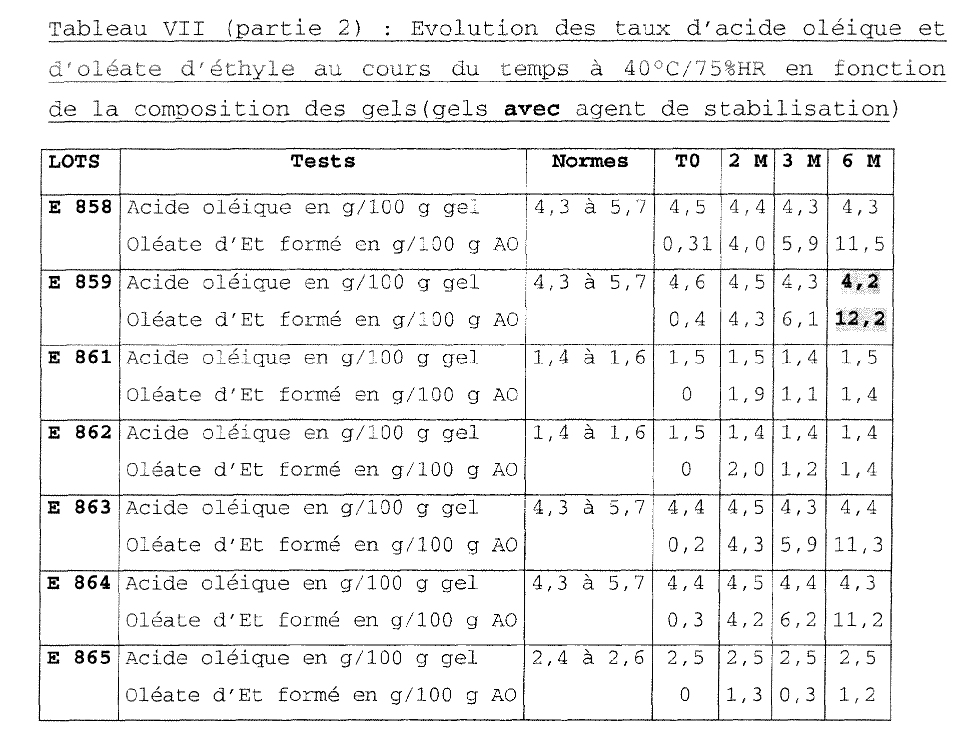

## Revendications

1. Composition pharmaceutique destinée à l'administration par voie transdermique ou par voie transmuqueuse d'au moins une substance active, et comprenant au moins un acide gras en tant que promoteur d'absorption percutanée, au moins un véhicule alcoolique, ainsi qu'au moins un agent stabilisant capable de stabiliser l'acide gras dans ladite composition pharmaceutique, dans laquelle le ou les agent(s) stabilisant(s) est(sont) sélectionné(s) dans le groupe constitué par les tampons et/ou les esters desdits acides gras.

2. Composition pharmaceutique selon la revendication 1 dans laquelle le ou les acides gras sont sélectionnés dans le groupe constitué par l'acide caprique, l'acide laurique, l'acide myristique, l'acide palmitique, l'acide stéarique, l'acide oléique, l'acide palmitoléique, l'acide linoléique, et l'acide linolénique.

3. Composition pharmaceutique selon l'une ou l'autre des revendications 1 et 2 présentant une teneur en poids en acides gras comprise entre 0,1% et 20%, de préférence entre 0,2% et 10%, et plus préférentiellement encore entre 0,5% et 5%.

4. Composition pharmaceutique selon l'une quelconque des revendications 1 à 3, présentant un pH entre 4 et 10, de préférence entre 5 et 9 et plus préférentiellement encore entre 6 et 8.

5. Composition pharmaceutique selon l'une quelconque des revendications 1 à 4, dans laquelle le ou les tampon(s) est(sont) sélectionné(s) dans le groupe constitué par :
- - les tampons alcalinisants ou basiques tels qu'un tampon phosphate (phosphate de sodium dibasique ou monobasique par exemple), un tampon citrate (citrate de sodium ou citrate de potassium par exemple), le carbonate de sodium, le bicarbonate de sodium, étant de préférence un mélange de carbonate de sodium et de bicarbonate de sodium, ou
- - les tampons neutres tels qu'un tampon Tris, de préférence un tampon phosphate.

6. Composition pharmaceutique selon l'une quelconque des revendications 1 à 5, présentant une teneur en poids en tampon(s) comprise entre 1 et 80%, de préférence entre 5 et 70% et plus préférentiellement encore entre 10 et 50%.

7. Composition pharmaceutique selon l'une quelconque des revendications 1 à 6, dans laquelle les esters des acides gras sont sélectionnés dans le groupe constitué par l'oléate d'éthyle, l'oléate d'isopropyle, le myristate d'isopropyle, le palmitate d'isopropyle, l'octanoate d'éthyle, le dodecanoate d'éthyle, le linoléate d'éthyle, et le linolénate d'éthyle.

8. Composition pharmaceutique selon la revendication 7, présentant une teneur en poids en ester(s) d'acides gras comprise entre 0,1 et 10%, de préférence entre 0,2 et 5% et plus préférentiellement encore entre 0,5 et 2,5%.

9. Composition pharmaceutique selon l'une quelconque des revendications 1 à 8, dans laquelle le(les) véhicule(s) alcoolique(s) est(sont) sélectionné(s) dans le groupe constitué par l'éthanol, et/ou l'isopropanol, étant de préférence l'éthanol.

10. Composition pharmaceutique selon la revendication 9 présentant une teneur en poids en véhzcule(s) alcaolique(s) comprise entre 10 et 90%, de préférence entre 20 et 80% et plus préférentiellement encore entre 40 et 70%.

11. Composition pharmaceutique selon l'une quelconque des revendications 1 à 10, comprenant un agent co-solvant sélectionné de préférence dans le groupe constitué par le glycérol (ou glycérine), le propylèneglycol et le polyéthylèneglycol, ainsi que leurs mélanges.

12. Composition pharmaceutique selon la revendication 11 présentant une teneur en poids en agent(s) co-solvant(s) comprise entre 0,5 et 20%, de préférence entre 1 et 10%.

13. Composition pharmaceutique selon l'une quelconque des revendications 1 à 12, se présentant sous la forme d'un gel et présentant une teneur en poids entre 0,2 et 30 % d'un agent gélifiant, de préférence entre 0,5 et 10 % et plus préférentiellement encore entre 0,3 et 5%.

14. Composition pharmaceutique selon l'une quelconque des revendications 1 à 13, dans laquelle la ou les substance(s) active(s) est(sont) sélectionnée(s) dans le groupe constitué par les oestrogènes, les progestatifs, les androgènes, les anti-oestragénes, les anti-andragénes au leurs mélanges.

15. Composition pharmaceutique selon la revendication 14 présentant une teneur en poids en substances actives comprise entre 0,01 et 5%.
